(19)

Europäisches
Patentamt
European
Patent Office
Office européen
des brevets

(11) **EP 2 743 677 A1**

(12) **EUROPEAN PATENT APPLICATION**

(43) Date of publication:
**18.06.2014 Bulletin 2014/25**

(51) Int Cl.:
***G01N 21/35*** (2014.01)   ***G01N 33/00*** (2006.01)
***B01J 20/22*** (2006.01)

(21) Application number: **12197184.0**

(22) Date of filing: **14.12.2012**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicant: **NXP B.V.**
**5656 AG Eindhoven (NL)**

(72) Inventors:
• **Soccol, Dimitri**
**Redhill, Surrey RH1 1NY (GB)**

• **Merz, Matthias**
**Redhill, Surrey RH1 1NY (GB)**

(74) Representative: **Crawford, Andrew et al**
**NXP SEMICONDUCTORS UK LTD**
**Intellectual Property & Licensing**
**Red Central**
**60 High Street**
**Redhill, Surrey RH1 1SH (GB)**

(54) **IR COx sensor and integrated circuit comprising the same**

(57)    An infrared $CO_x$ sensor (10) is disclosed comprising an infrared source (14), an infrared detector (16) and an optical path between the infrared source and the infrared detector exposed to the environment of said sensor, wherein the optical path comprises a $CO_x$-binding medium (20, 20'), said medium being substantially transparent to infrared radiation having a wave number in the range of 2,000-2,500 cm$^{-1}$.

**FIG. 1**

**Description**

FIELD OF THE INVENTION

The present invention relates to an infrared $CO_x$ sensor such as a $CO_2$ sensor comprising an infrared source, an infrared detector and an optical path between the infrared source and the infrared detector and exposed to the environment of said sensor.

BACKGROUND OF THE INVENTION

Infrared (IR) sensors for detecting CO and $CO_2$ are well-known. Such sensors are commonly used because of the excellent selectivity of such sensors due to the fact that $CO_2$ has a strong and characteristic IR absorption at around 2340 $cm^{-1}$ and 2365 $cm^{-1}$ attributable to the asymmetric C=O stretch vibration in the $CO_2$ molecules, whereas the C=O stretch vibration of CO can be found around 2143 $cm^{-1}$.

An example of such a sensor is the ICx Photonics sensor chip™ $CO_2$ sensor 2, as marketed by FLIR® Systems Inc. It is particularly desirable to integrate such a sensor on a chip or integrated circuit (IC), as the miniaturization of the sensor enables sensing applications in a wide variety of application domains, e.g. to predict the remaining shelf life of the product, e.g. perishable food stuff. The $CO_2$ sensor may for instance be adapted to determine changes in the $CO_2$ content of the ambient atmosphere. Alternatively, the gas sensor may be used to detect changes in the gas composition of larger environment such as buildings or may be used in medical application domains, e.g. in breathing apparatuses.

With the ongoing diversification of electronic devices or electronic information gathering such as by RF tags on packaged articles, it is often desirable to include different types of sensors in a single IC. For instance, the detection of other environmental parameters, for instance temperature and humidity such as for HVAC (heating, ventilation and air conditioning) control in buildings and cars, are particularly desirable in certain application domains. In addition, sensing of analytes of interest, e.g. $CO_2$, may be desirable in such application domains. However, it is difficult to manufacture $CO_2$ sensors having the desired sensitivity in a cost-effective manner. In particular, IR $CO_2$ sensors require a relatively large optical path between the IR source and IR detector. For instance, in the aforementioned ICx Photonics sensor chip™ $CO_2$ sensor 2, an optical path length of approximately 5 cm is required to obtain a $CO_2$ sensor having the desired sensitivity. This is because a minimum number of $CO_2$ molecules are needed to attenuate the infrared signal to a measurable level. For instance, for a partial pressure of 30 ppm $CO_2$ (3 Pa $CO_2$), this corresponds to a $CO_2$ molar density of 1.25 mmole $CO_2$ per $m^3$ according to the ideal gas law:

$$\text{Molar density (gas)} = n/V = p(P/RT)$$

with n the number of moles of $CO_2$ molecules, V the volume of gas, p the pressure [ppm], P the standard pressure of 101.325 kPa, R the universal gas constant [J/mole/K] and T the temperature [K]. The required minimum optical path length makes such sensors relatively bulky, which for instance complicates or even precludes the integration of multiple sensors on a single IC or chip.

SUMMARY OF THE INVENTION

The present invention seeks to provide a more compact IR $CO_x$ sensor according to the opening paragraph.
The present invention further seeks to provide an IC comprising such a sensor.
According to an aspect of the present invention, there is disclosed an infrared $CO_x$ sensor comprising an infrared source, an infrared detector and an optical path between the infrared source and the infrared detector and exposed to the environment of said sensor, wherein the optical path comprises a $CO_x$-binding medium, said medium being substantially transparent to infrared radiation having a wave number in the range of 2,000-2,500 $cm^{-1}$.
The present invention is based on the insight that certain media, e.g. solids or liquids, can adsorb $CO_x$, e.g. CO or $CO_2$, without chemically reacting with the gas such that the characteristic IR absorption bands remain located at e.g. around 2340 $cm^{-1}$ and 2365 $cm^{-1}$ for $CO_2$ and 2143 $cm^{-1}$ for CO. As such media are transparent to IR radiation in this wave number range, an accurate detection of the COx-concentration in the environment or atmosphere to which the sensor is exposed can be achieved. More particularly, such solids or liquids can bind $CO_x$ to such an extent that these media exhibit an increased $CO_x$ density compared to air, such that the optical path length between the IR source and the IR detector can be reduced compared to IR $CO_2$ sensors such as the ICx Photonics sensor chip™ $CO_2$ sensor 2, which utilize an air-filled optical path, thus yielding a more compact $CO_x$ sensor.
In a preferred embodiment, the infrared source and the infrared detector are adjacent to each other at a first end of the optical path, the infrared $CO_x$ sensor further comprising a reflective surface at a second end of the optical path opposite said first end for reflecting infrared radiation from the infrared source to the infrared detector. This doubles the effective length of the optical path as the IR radiation travels through the medium twice from the infrared source to the infrared detector, thus further reducing the required minimum length of the optical path.

In an embodiment, the optical path has a path length in the range of 2-10 mm, which yields a particularly compact $CO_x$ sensor.

The infrared $CO_x$ sensor may further comprise a gas-permeable cap at the second end of the optical path, wherein the reflective surface is attached to a surface of the gas-permeable cap facing the optical path. This yields a sensor of relatively simple design, which therefore can be manufactured in a cost-effective manner.

The $CO_x$-binding medium may be a solid or liquid. In case the medium is a liquid, the infrared $CO_x$ sensor may further comprise a container surrounding the optical path for containing said liquid.

In an embodiment, the medium comprises a solid portion, wherein the optical path preferably comprises a clearance between the solid portion and at least one of the infrared source and the infrared detector. This has the advantage of reducing the critical diffusion length of the $CO_x$ molecules into the medium, which improves the response time of the sensor. However, such a clearance may be omitted from the design of the IR $CO_x$ sensor of the present invention.

The solid portion may be attached to the reflective surface, which simplifies the manufacturing of the sensor, thus reducing manufacturing cost.

The solid portion may comprise a metal-organic framework (MOF) such as copper(II)-benzene-1,3,5-tricarbo-xylate ($Cu_3BTC_2$), Zn-MOF-74, aluminium-benzene-1,3,5-tricarboxylate (Al-BTC), chromium (III)-benzene-1,4-dicarbo-xylate ($Cr_2BDC_3$), Aluminium-OH- benzene-1,4-dicarboxylate (Al-OH-BDC) and ZIF-8, or any other suitable MOF, as such a MOF has a particularly high $CO_x$, e.g. $CO_2$, molar density, which therefore yields a particularly sensitive IR $CO_x$ sensor, such that a particularly short optical path may be selected.

The average particle size of the metal-organic framework preferably is less than 2 micron to avoid dispersive reflection of the IR radiation.

The metal-organic framework may be incorporated in a polymer matrix to facilitate immobilization of the MOF in the sensor, as MOFs typically are powders, which may be cumbersome to immobilize otherwise.

Such a polymer matrix preferably comprises a polymer selected from the group consisting of PIM-1, polyimide (PI), a fluorinated polyimide such as 6FDA-TrMPD, polytetrafluoroethylene (PTFE), polysulfone (PSU), polylactic acid (PLA), poly-(ethylene oxide) (PEO) and polydimethylsiloxane (PDMS), as such polymers are relatively porous and also have an affinity to bind $CO_x$, e.g. $CO_2$, thus further enhancing the $CO_x$ binding ability of the medium.

Alternatively, the solid medium portion may exclude the MOF and may simply comprise a polymer such as PIM-1, polyimide (PI), a fluorinated polyimide such as 6FDA-TrMPD, polytetrafluoroethylene (PTFE), polysulfone (PSU), polylactic acid (PLA), poly-(ethylene oxide) (PEO) and polydimethylsiloxane (PDMS), as such polymers also have an affinity to bind $CO_x$, e.g. $CO_2$ as mentioned before.

In case the medium is a liquid, the liquid may be comprise a solvent selected from the group of solvents including polyethylene glycol 300, polyethylene glycol 200, tetrahydrofuran, methyl acetate, 2-butanone, acetone, 1,4 dioxane, glycerol carbonate, ethyl acetate and propyl acetate or combinations thereof as these solvents have a particularly high affinity to bind $CO_x$, e.g. $CO_2$.

Preferably, at least one of the infrared source and the infrared detector comprises a bandpass filter for passing infrared radiation having a wave number in the range of 2,000-2,500 cm$^{-1}$ such that only relevant IR radiation is passed into and/or received from the optical path.

The infrared $CO_x$ sensor according to one or more embodiments of the present invention may be integrated into an IC or chip, which facilitates utilization of the sensor in application domains where its small form factor is beneficial, e.g. as RF tags on packaged articles, HVAC (heating, ventilation and air conditioning) control in buildings and cars and so on. In addition, the infrared $CO_x$ sensor according to one or more embodiments of the present invention may be manufactured at lower cost than the state of the art infrared $CO_x$ sensors.

BRIEF DESCRIPTION OF THE EMBODIMENTS

Embodiments of the invention are described in more detail and by way of non-limiting examples with reference to the accompanying drawings, wherein:

FIG. 1 schematically depicts a non-dispersive IR $CO_x$ sensor according to an embodiment of the present invention; and

FIG. 2 schematically depicts a non-dispersive IR $CO_x$ sensor according to another embodiment of the present invention.

DETAILED DESCRIPTION OF THE DRAWINGS

It should be understood that the Figures are merely schematic and are not drawn to scale. It should also be understood that the same reference numerals are used throughout the Figures to indicate the same or similar parts.

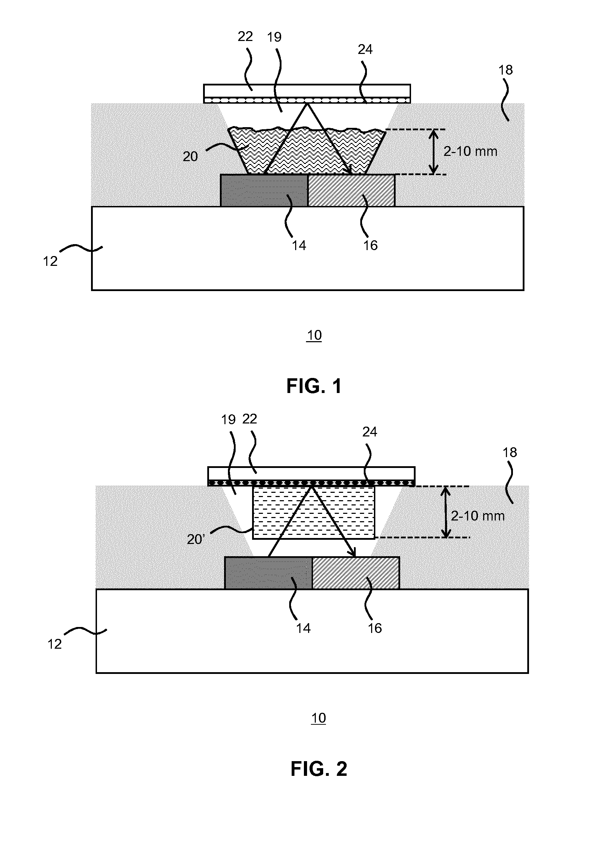

FIG. 1 schematically depicts a first embodiment of a non-dispersive $CO_x$ sensor 10 of the present invention. The sensor 10 comprises a carrier 12 with an IR source 14 and an IR detector 16 adjacent to the IR source 14 formed thereon. The IR source 14 and IR detector 16 may be any suitable known IR source and IR detector. Although the IR source 14 and IR detector 16 are shown as different components, it should be understood that it is equally feasible that the IR source 14 and IR detector 16 are integrated into a single component.

The carrier 12 may be any suitable material. In an embodiment, the carrier 12 is a substrate of an IC, which may be any suitable substrate material e.g. a silicon substrate, a silicon-on-insulator substrate, a GaN substrate and so on. Alternatively, the sensor 10 may be integrated heterogeneously, e.g. using separate or discrete components on a PCB as substrate. Although in FIG. 1 the IR source 14 and IR detector 16 are shown directly on the substrate 10, it should be understood that it is equally feasible that the IR source 14 and IR detector 16 are separated from the substrate 12 by one or more layers, e.g. a metallization stack comprising one or more patterned metal layers spatially separated by one or more electrically insulating layers, in which case the IR source 14 and IR detector 16 may be formed on the passivation layer(s) covering the metallization stack. In this embodiment, the IR source 14 and IR detector 16 may be connected to circuitry on the substrate 12 by respective portions of the metallization stack. At least one of the IR source 14 and IR detector 16 may comprise a band pass filter that allows IR radiation of the appropriate wave number, e.g. 2,000-2,500 cm$^{-1}$ to pass the filter.

The $CO_x$ sensor 10 further comprises a layer 18 over the carrier or substrate 12, in which a cavity 19 is formed in which the IR source 14 and IR detector 16 are exposed. In an embodiment, the layer 18 may be a dielectric layer such as an oxide or nitride layer, in which the cavity 19 may be formed using any suitable etch recipe. In an alternative embodiment, the layer 18 may be a polymer layer such as a polyimide layer, in which the cavity 19 may be formed by selective exposure of the polymer layer to light having an appropriate wavlength through a photolithographic mask followed by a development step, or by any other suitable patterning step, e.g. etching.

In an alternative embodiment in which the sensor is integrated heterogeneously as previously explained, the layer 18 may be a polymer layer, which for instance may consist of a molding compound. Such a molding compound may for instance be formed in an open cavity molding process that leaves an open space on top of the IR source 14 and detector 16 mounted on a PCB 12.

The cavity 19 comprises a liquid 20 that is transparent to IR radiation having a wave number of 2,000-2,500 cm$^{-1}$, more preferably to IR radiation having a wave number of 2,300-2,400 cm$^{-1}$ and that has an affinity for binding $CO_x$, e.g. CO or $CO_2$, as will be explained in more detail later. The solvents may be placed in the cavity 19 in pure form or may comprise any suitable solute. The cavity 19 is sealed by a gas-permeable cap 22 such as a porous or perforated solid lid or a gas-permeable membrane that has a reflective film 24 adhered to its surface facing the IR source 14 and the IR detector 16, such that IR radiation emitted by the IR source 14 is reflected by the reflective film 24 and redirected towards the IR detector 16, as indicated by the arrow in FIG. 1. The gas-permeable cap 22 may be fixated to the layer 18 in any suitable manner, e.g. using adhesives or other fixing means.

The reflective film 24 is also gas-permeable. This may for instance be achieved by limiting the thickness of the reflective film 24, or by using a material for the reflective film 24 that is intrinsically gas-permeable or has been made gas-permeable by forming holes in the reflective film 24, e.g. through perforation. The reflective film 24 may be an aluminium film or a gold film although other suitable materials will be apparent to the skilled person.

The length of the optical path between the IR source 14 and the IR detector 16 is defined by the thickness of the liquid 20 in the cavity 19, as this is the medium containing the $CO_x$ molecules that interact with the IR radiation in the wave number range of 2,000 - 2,500 cm$^{-1}$ from the IR source 14. The thickness of the layer of the medium 20 is governed by the ability of the medium 20 to bind $CO_x$, as the thickness should be sufficient to ensure that the sensor 10 has the desired detection sensitivity. More specifically, the sensitivity of the sensor 10 is directly proportional to the molar density ratio $M_R$ of $CO_x$ in the medium 20, such that the $CO_x$ detection limit of the sensor 10 is proportional to $M_R.OL$, with OL being the optical path length of the IR $CO_x$ sensor 10.

The solubility of a gas such as CO or $CO_2$ in a liquid or solid medium may be approximated using Henry's Law: $p = k_H.x$ in which p is the pressure in atmosphere, $k_H$ is the Henry constant and x is the molar fraction of the gas in the material. Although this law only provides a first order approximation that becomes inaccurate at higher partial pressures of the gas, it has been found that the approximation is appropriate for the concentration ranges of $CO_x$ that are typically measured by $CO_x$ sensors, e.g. up to 1% $CO_x$, e.g. $CO_2$.

The molar fraction of $CO_2$ can be easily recalculated into a molar density when the molecular weight and mass density of the dissolving compound are known. The ratio of molar densities of the binding medium 20 and the bound $CO_x$ gas may be expressed by the following formula:

$$ratio = \left(\frac{1}{k_H}\frac{\rho}{M_w}\right)\Big/\frac{P}{RT}$$

With $\rho$ the mass density [kg/m$^3$] and $M_w$ the molecular weight [kg/mole] of the medium 20.

Table 1 provides a non-exhaustive list of suitable solvents to be used as the medium 20. These solvents have in common that they all are transparent to IR radiation having a wave number in the range of 2,000 - 2,500 cm$^{-1}$.

Table 1 Non-exhaustive list of solvents that show an increase in $CO_2$ molar density compared to the $CO_2$ molar density in air. The ratio of increase is shown in the last column. The reference value in air for 30 ppm $CO_2$ is 1.23e-03 mol/m$^3$.

| | $k_H(CO_2)$ | $M_w$ | Density | Molar density | Molar density | Ratio |
|---|---|---|---|---|---|---|
| | | (solvent) | (solvent) | (solvent) | ($CO_2$) | |
| | [atm] | [kg/mole] | [kg/m$^3$] | [mol/m$^3$] | $CO_2$/m$^3$] | |
| **Solvents with > 5 times molar density increase:** | | | | | | |
| PEG 300 | 10.87 | 3.00E-01 | 1124 | 3.75E+03 | 1.03E-02 | 8.43 |
| PEG 200 | 16.42 | 2.00E-01 | 1124 | 5.62E+03 | 1.03E-02 | 8.37 |
| THF | 37.04 | 7.21 E-02 | 889 | 1.23E+04 | 9.99E-03 | 8.14 |
| methyl acetate | 44.39 | 7.41 E-02 | 932 | 1.26E+04 | 8.50E-03 | 6.93 |
| MEK (2-butanone) | 40.92 | 7.21 E-02 | 805 | 1.12E+04 | 8.19E-03 | 6.67 |
| acetone | 50.33 | 5.81 E-02 | 791 | 1.36E+04 | 8.12E-03 | 6.62 |
| 1,4-dioxane | 44.01 | 8.81 E-02 | 1033 | 1.17E+04 | 7.99E-03 | 6.52 |
| glycerol carbonate | 47.17 | 1.18E-01 | 1400 | 1.19E+04 | 7.54E-03 | 6.15 |
| ethyl acetate | 43.48 | 8.81 E-02 | 897 | 1.02E+04 | 7.02E-03 | 5.73 |
| propyl acetate | 41.17 | 1.02E-01 | 890 | 8.71 E+03 | 6.35E-03 | 5.18 |
| **Solvents with 3-5 times molar density increase:** | | | | | | |
| PEG 600 | 9.53 | 6.00E-01 | 1126 | 1.88E+03 | 5.91 E-03 | 4.82 |
| amyl acetate | 35.71 | 1.30E-01 | 876 | 6.73E+03 | 5.65E-03 | 4.61 |
| isobutyl acetate | 40.00 | 1.16E-01 | 875 | 7.53E+03 | 5.65E-03 | 4.61 |
| DEGDME | 41.79 | 1.34E-01 | 937 | 6.98E+03 | 5.01 E-03 | 4.09 |
| n-methylpyrrolido ne | 62.56 | 9.91 E-02 | 1028 | 1.04E+04 | 4.97E-03 | 4.06 |
| PEGDME 150 | 45.84 | 1.50E-01 | 1089 | 7.26E+03 | 4.75E-03 | 3.87 |
| propylene carbonate | 78.21 | 1.02E-01 | 1205 | 1.18E+04 | 4.53E-03 | 3.69 |
| n-formyl morpholine | 67.80 | 1.15E-01 | 1145 | 9.95E+03 | 4.40E-03 | 3.59 |
| TEGDME | 0.04 | 1.78E+0 2 | 986 | 5.53E+00 | 4.34E-03 | 3.53 |
| methanol | 178.39 | 3.20E-02 | 788 | 2.46E+04 | 4.14E-03 | 3.37 |
| PEGDME 250 | 0.03 | 2.50E+0 2 | 1089 | 4.36E+00 | 4.08E-03 | 3.33 |

(continued)

| Solvents with 3-5 times molar density increase: | | | | | |
|---|---|---|---|---|---|
| tributyl phosphate | 28.17 | 2.66E-01 | 973 | 3.65E+03 | 3.89E-03 | 3.17 |
| sulfolane | 87.98 | 1.20E-01 | 1261 | 1.05E+04 | 3.58E-03 | 2.92 |
| **Solvents with < 3 times molar density increase:** | | | | | |
| tetraglyme | 41.25 | 2.22E-01 | 1011 | 4.55E+03 | 3.31 E-03 | 2.70 |
| [Bmim][NTf$_2$] | 33.40 | 4.19E-01 | 1440 | 3.43E+03 | 3.08E-03 | 2.51 |
| [Hmim][FAP] | 25.20 | 6.12E-01 | 1560 | 2.55E+03 | 3.03E-03 | 2.47 |
| perfluoroheptan e | 47.89 | 3.88E-01 | 1745 | 4.50E+03 | 2.82E-03 | 2.30 |
| methyl oleate | 37.17 | 2.96E-01 | 874 | 2.95E+03 | 2.38E-03 | 1.94 |
| butyl oleate | 35.84 | 3.39E-01 | 871 | 2.57E+03 | 2.15E-03 | 1.76 |

A preferred selection from this table includes the solvents that exhibit at least a three-fold increase in the $CO_2$ molar density compared to air. A more preferred selection from this table includes the solvents that exhibit at least a fivefold increase in the $CO_2$ molar density compared to air. The data in Table 1 is known per se; see for instance W. Kunerth in Phys. Rev. 19, pages 512-524 (1922); M.B. Miller et al. in J. Chem. Eng. Data, 56, pages 1565-1572 (2011) and O. Aschenbrenner et al. in Energy Environ. Sci. 3, pages 1106-1113 (2010). However, it should be understood that these publications do not disclose or suggest the ratio in the rightmost column of Table 1; this ratio has been recognized and determined by the present inventors.

FIG. 2 depicts an alternative embodiment of the non-dispersive COx sensor 10 or the present invention, in which the liquid medium 20 has been replaced by a solid medium 20'. The solid medium 20' may be fixated to the gas-permeable lid 22, e.g. using an adhesive, or may be placed in the cavity 19 instead. The fixating of the solid medium 20' to the gas-permeable lid 22 has the advantage that no deposition of the solid medium 20' in the cavity 19 is required, which simplifies the manufacturing process of the sensor 10.

The solid medium 20' preferably does not completely fill the cavity 19 to minimize the critical diffusion length of the gas molecules into the medium 20'. For instance, a clearance may exist between the medium 20' and the carrier 12 as shown in FIG. 2. Alternatively, in case the medium 20' is deposited in the cavity 19, a clearance may exist between the medium 20' and the reflective film 24. In yet another embodiment, the solid medium may be a (solid) porous medium such as a foam to limit the critical diffusion length of the gas molecules into the medium.

In yet another embodiment, the critical diffusion length of the gas molecules into the medium is limited by a clearance between the reflective film 24 and the walls of the cavity 19 to create a lateral passage way for the gas to reach the medium 20'. This may for instance be achieved by a stripe of (aluminum) foil that is narrower than the cavity opening. In this embodiment, it is therefore not necessary for the reflective film to be gas-permeable.

In order to avoid excessive dispersive reflection of the IR radiation, the solid medium 20' preferably has a surface roughness of less than 2 micron. Table II provides a non-exhaustive list of suitable solids to be used as the medium 20'. These solvents have in common that they all are transparent to IR radiation having a wave number in the range of 2,000 - 2,500 cm$^{-1}$.

Table 1. Non-exhaustive list of polymers and metal-organic frameworks that show an increase in $CO_2$ molar density compared to the $CO_2$ molar density in air. The ratio of increase is shown in the last column. The reference value in air for 30 ppm of $CO_2$ is 1.23e-03 mol/m$^3$.

| | k$_H$(CO$_2$) | M$_w$ | Density | Molar density | Molar density | Ratio |
|---|---|---|---|---|---|---|
| | | (solid) | (solid) | (solid) | (CO$_2$) | |
| | [atm] | [kg/mole] | [kg/m$^3$] | [mol/m$^3$] | [mol CO$_2$/m$^3$] | |
| **Polymers:** | | | | | | |
| PIM-1 | 2.52 | 4.61 E-01 | 1124 | 2.44E+03 | 2.91 E-02 | 23.72 |
| PI4 | 2.90 | 5.58E-01 | 1352 | 2.42E+03 | 2.51 E-02 | 20.46 |

(continued)

| | $k_H(CO_2)$ | $M_w$ | Density | Molar density | Molar density | Ratio |
|---|---|---|---|---|---|---|
| | | (solid) | (solid) | (solid) | ($CO_2$) | |
| | [atm] | [kg/mole] | [kg/m$^3$] | [mol/m$^3$] | [mol $CO_2$/m$^3$] | |
| **Polymers:** | | | | | | |
| PTFE (1) | 59.20 | 4.88E-02 | 1750 | 3.58E+04 | 1.82E-02 | 14.81 |
| PSU | 5.65 | 4.70E-01 | 1250 | 2.66E+03 | 1.41 E-02 | 11.51 |
| PLA | 0.07 | 9.00E+01 | 1320 | 1.47E+01 | 6.08E-03 | 4.96 |
| PTFE (2) | 258.84 | 4.88E-02 | 1750 | 3.58E+04 | 4.15E-03 | 3.39 |
| PEO | 271.80 | 4.41 E-02 | 1219 | 2.77E+04 | 3.05E-03 | 2.49 |
| PDMS | 196.38 | 9.42E-02 | 970 | 1.03E+04 | 1.57E-03 | 1.28 |
| | | | | | | |
| **Metal-organic frameworks:** | | | | | | |
| $Cu_3BTC_2$ (HKUST-1) | 1.10 | 2.02E-01 | 350 | 1.74E+03 | 4.74E-02 | 38.69 |
| Zn (MOF-74) | 0.00 | 2.61 E+02 | 350 | 1.34E+00 | 4.30E-02 | 35.10 |
| Al-BTC (MIL-110) | 1.78 | 2.34E-01 | 350 | 1.50E+03 | 2.52E-02 | 20.54 |
| $Cr_2BDC_3$ (MIL-101) | 0.93 | 5.96E-01 | 350 | 5.87E+02 | 1.89E-02 | 15.41 |
| Al-OH-BDC (MIL-53) | 3.00 | 2.08E-01 | 400 | 1.92E+03 | 1.92E-02 | 15.65 |
| ZIF-8 | 4.15 | 2.30E-01 | 350 | 1.52E+03 | 1.10E-02 | 8.99 |

The data in Table 2 is known per se; see for instance: O. Hölck, "Gas Sorption and Swelling in Glassy Polymers" PhD thesis (2008); A. Kasturirangan, "Specific interactions in carbon dioxide + polymer systems" PhD thesis (2007); J. Liu, "Adsorption Equilibrium and Mass Transfer in Metal-Organic Frameworks and Adsorption on Carbon Surfaces in the Henry's Law Region", PhD thesis (2011); S. Bordiga, L. Regli, F. Bonino, E. Groppo, C. Lamberti, B. Xiao, P. S. Wheatley, R. E. Morris and A. Zecchina "Adsorption properties of HKUST-1 toward hydrogen and other small molecules monitored by IR" Phys chem chem phys 9 pp. 2676 - 2685 (2007); S. Marx, W. Kleist, A. Baiker, "Synthesis, structural properties, and catalytic behavior of Cu-BTC and mixed-linker Cu-BTC-PyDC in the oxidation of benzene derivatives" J. Catal. 281 pp. 76 - 87 (2011); Y. C. Pan, Y. Y. Liu, G. F. Zeng, L. Zhao, Z. P. Lai, "Rapid Synthesis of Zeolitic Imidazolate Framework-8 (ZIF-8) Nanocrystals in Aqueous System" Chem. Commun. 47 pp. 2071 - 2073 (2011); and M. S. Y. Parast, A. Morsali, "Synthesis and characterization of porous Al(III) metal-organic framework nanoparticles as a new precursor for preparation of Al2O3 Nanoparticles" Inorg. Chem. Commun. 14 pp. 645 - 648 (2011). However, it should be understood that these publications do not disclose or suggest the ratio in the rightmost column of Table II; this ratio has been recognized and determined by the present inventors.

The solid materials in Table 2 have in common that they all are transparent to IR radiation having a wave number in the range of 2,000 - 2,500 cm$^{-1}$. The listed polymers not only have a high molar density ratio, but are rubberlike polymers that have a relatively large free volume, which shortens the diffusion time of the $CO_x$ molecules into the polymer, which therefore reduces the time it takes for the $CO_x$ concentration in the polymer to reach equilibrium, thus improving response times of the IR $CO_x$ sensor 10. The polymers may be processed in any suitable manner.

The metal-organic frameworks (MOFs) are particularly interesting due to their particularly high molar density ratio and their porous nature, which shortens the diffusion time of the $CO_x$ molecules into the polymer, which therefore reduces the time it takes for the $CO_x$ concentration in the polymer to reach equilibrium, thus improving response times of the IR $CO_x$ sensor 10. The MOFs typically are powders. In an embodiment, in order to facilitate the immobilization of the MOF on the gas-permeable lid 22, the MOF may be trapped in any suitable polymer matrix. In a preferred embodiment, the polymer matrix is formed by a polymer from Table II such that both the MOF and the polymer matrix have an increased ability to contain $CO_x$, e.g. $CO_2$, compared to air. Examples of such mixed matrix materials have for instance been disclosed by S. Bordiga et al. in Phys. chem. chem. phys., 9, pages 2676-2685 (2007).

Preferably, the MOF powder particles comprise an average particle size of less than 2 micron to avoid excessive scattering of IR radiation. This is particularly relevant when the MOF is trapped in a polymer matrix, as turbidity by IR scattering can occur at the particle-polymer interface.

At this point, it is noted that the materials in Table I and Table II have in common that they exhibit an increased molar density of $CO_x$, e.g. $CO_2$, compared to air, which means that a higher density of $CO_x$ molecules is present per unit length in the optical path of the IR $CO_x$ sensor 10, such that the optical path length of the IR $CO_x$ sensor 10 of the present invention can be reduced compared to an IR sensor having air as a medium in its optical path by a factor governed by the ratio in Table I and II; i.e. $OL = OL_{air}/Ratio$, in which OL is the minimum optical path length of the IR $CO_x$ sensor 10 of the present invention and $OL_{air}$ is the minimum optical path length of an the IR $CO_x$ sensor using air as the medium in its optical path.

For the compounds in Table 1 and 2, this translates to a minimum optical path length in the range of 2-10 mm as indicated in FIG. 1 and 2 that is required to ensure that the IR $CO_2$ sensor 10 of the present invention has a sensitivity that is comparable to an IR $CO_2$ sensor having an air-filled optical path with a path length of 5 cm. It will be immediately apparent that the choice of a liquid medium 20 from Table I or a solid medium 20' from Table II as the optical path medium in the IR $CO_x$ sensor 10 of the present invention yields a significantly more compact sensor compared to IR $CO_2$ sensors having an air-filled optical path.

FIG. 1 and 2 schematically depict a preferred embodiment of the present invention in which the IR source 14 and the IR detector 16 are laterally displaced, e.g. located adjacent to each other, on the carrier 12, as in this embodiment the IR radiation must travel through the medium 20 or 20' twice, thus doubling the effective optical path length of the IR $CO_x$ sensor 10. However, it should be understood that alternative arrangement, e.g. an arrangement in which the IR source 14 and the IR detector 16 are not located in the same plane but for instance are located opposite to each other with the medium 20 or 20' placed between the IR source 14 and the IR detector 16 is also feasible.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements. The invention can be implemented by means of hardware comprising several distinct elements. In the device claim enumerating several means, several of these means can be embodied by one and the same item of hardware. The mere fact that certain measures are recited in mutually different dependent claims does not indicate that a combination of these measures cannot be used to advantage.

## Claims

1. An infrared $CO_x$ sensor (10) comprising an infrared source (14), an infrared detector (16) and an optical path between the infrared source and the infrared detector exposed to the environment of said sensor, wherein the optical path comprises a $CO_x$-binding medium (20, 20'), said medium being substantially transparent to infrared radiation having a wave number in the range of 2,000-2,500 cm$^{-1}$.

2. The infrared $CO_x$ sensor (10) of claim 1, wherein the infrared source (14) and the infrared detector (16) are adjacent to each other at a first end of the optical path, the infrared $CO_x$ sensor further comprising a reflective surface (24) at a second end of the optical path opposite said first end for reflecting infrared radiation from the infrared source to the infrared detector.

3. The infrared $CO_x$ sensor (10) of claim 2, further comprising a gas-permeable cap (22) at the second end of the optical path, wherein the reflective surface (24) is attached to a surface of the gas-permeable cap facing the optical path.

4. The infrared $CO_x$ sensor (10) of any of claims 1-3, wherein the $CO_x$-binding medium is porous.

5. The infrared $CO_x$ sensor (10) of any of claims 1-4, wherein the medium (20') comprises a solid portion, and wherein the optical path preferably comprises a clearance between the solid portion and at least one of the infrared source (14) and the infrared detector (16).

6. The infrared $CO_x$ sensor (10) of claim 5, wherein the solid portion (20') is attached to the reflective surface (24).

**7.** The infrared CO$_x$ sensor (10) of claim 5 or 6, wherein the solid portion (20') comprises a metal-organic framework such as copper(II)-benzene-1,3,5-tricarboxylate (Cu$_3$BTC$_2$), Zn MOF-74, aluminium- benzene-1,3,5-tricarboxylate (Al-BTC), chromium (III)-benzene-1,4-dicarboxylate (Cr$_2$BDC$_3$), Aluminium-OH-benzene-1,4-dicarboxylate (Al-OH-BDC) and ZIF-8.

**8.** The infrared CO$_x$ sensor (10) of claim 7, wherein the average particle size of the metal-organic framework is less than 2 micron.

**9.** The infrared CO$_x$ sensor (10) of claim 7 or 8, wherein the metal-organic framework is incorporated in a polymer matrix.

**10.** The infrared CO$_x$ sensor (10) of claim 9, wherein the polymer matrix comprises a polymer selected from the group consisting of PIM-1, polyimide (PI), polytetrafluoroethylene (PTFE), polysulfone (PSU), polylactic acid (PLA), poly-(ethylene oxide) (PEO) and polydimethylsiloxane (PDMS).

**11.** The infrared CO$_x$ sensor (10) of claim 5 or 6, wherein the solid portion (20') comprises a polymer such as PIM-1, polyimide (PI), polytetrafluoroethylene (PTFE), polysulfone (PSU), polylactic acid (PLA), poly-(ethylene oxide) (PEO) and polydimethylsiloxane (PDMS).

**12.** The infrared CO$_x$ sensor (10) of any of claims 1-4, wherein the medium (20) is a liquid, and wherein the infrared CO$_x$ sensor further comprises a container (18) surrounding the optical path for containing said liquid.

**13.** The infrared CO$_x$ sensor (10) of claim 12, wherein the liquid comprises a solvent selected from the group of solvents including polyethylene glycol 300, polyethylene glycol 200, tetrahydrofuran, methyl acetate, 2-butanone, acetone, 1,4 dioxane, glycerol carbonate, ethyl acetate and propyl acetate or combinations thereof.

**14.** The infrared CO$_x$ sensor (10) of any of claims 1-13, wherein at least one of the infrared source (14) and the infrared detector (16) comprises a bandpass filter for passing infrared radiation having a wave number in the range of 2,000-2,500 cm$^{-1}$.

**15.** An integrated circuit comprising the infrared CO$_x$ sensor (10) of any of claims 1-14.

10

FIG. 1

10

FIG. 2

EP 2 743 677 A1

**EUROPEAN SEARCH REPORT**

Application Number

EP 12 19 7184

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/028846 A1 (YAGHI OMAR M [US] ET AL) 2 February 2012 (2012-02-02) | 1,4,7-11 | INV.<br>G01N21/35 |
| Y | * [0008], [0064], [0065], [0069]; figure 1 * | 2,3,5,6,12-15 | G01N33/00<br>B01J20/22 |
| Y | WO 2006/111433 A1 (BOSCH GMBH ROBERT [DE]; LUDWIG RONNY [DE]; SAUER MAXIMILIAN [DE]) 26 October 2006 (2006-10-26)<br>* [0002], [0025]-[0027]; figure 1 * | 2,3,5,6,15 | |
| Y | DE 102 00 908 A1 (WIEGLEB GERHARD [DE]) 31 July 2003 (2003-07-31)<br>* figure 1 * | 2,3,6,14,15 | |
| Y | PASQUALI I ET AL: "Measurement of CO2 sorption and PEG 1500 swelling by ATR-IR spectroscopy",<br>JOURNAL OF SUPERCRITICAL FLUIDS, PRA PRESS, US,<br>vol. 45, no. 3, 1 July 2008 (2008-07-01), pages 384-390, XP022679472,<br>ISSN: 0896-8446, DOI:<br>10.1016/J.SUPFLU.2008.01.015<br>[retrieved on 2008-02-09]<br>* figure 1 * | 12,13 | |
| A | KRISTI L. KAUFFMAN ET AL: "Selective Adsorption of CO2 from Light Gas Mixtures by Using a Structurally Dynamic Porous Coordination Polymer",<br>ANGEWANDTE CHEMIE INTERNATIONAL EDITION,<br>vol. 50, no. 46,<br>11 November 2011 (2011-11-11), pages 10888-10892, XP055065963,<br>ISSN: 1433-7851, DOI:<br>10.1002/anie.201104130<br>* figure 2 * | 1-15 | TECHNICAL FIELDS SEARCHED (IPC)<br><br>G01N<br>B01J |

-/--

The present search report has been drawn up for all claims

| Place of search | Date of completion of the search | Examiner |
|---|---|---|
| Munich | 10 June 2013 | Mason, William |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

11

Europäisches Patentamt

European Patent Office

Office européen des brevets

# EUROPEAN SEARCH REPORT

**Application Number**

EP 12 19 7184

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | JEFFREY T. CULP ET AL: "Mechanism for the Dynamic Adsorption of $CO_2$ and $CH_4$ in a Flexible Linear Chain Coordination Polymer as Determined from In Situ Infrared Spectroscopy", JOURNAL OF PHYSICAL CHEMISTRY C, vol. 114, no. 5, 11 February 2010 (2010-02-11), pages 2184-2191, XP055065964, ISSN: 1932-7447, DOI: 10.1021/jp908202s * figures 1-2 * | 1-15 | |
| | | | **TECHNICAL FIELDS SEARCHED (IPC)** |

The present search report has been drawn up for all claims

| Place of search | Date of completion of the search | Examiner |
|---|---|---|
| Munich | 10 June 2013 | Mason, William |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 12 19 7184

This annex lists the patent family members relating to the patent documents cited in the above-mentioned European search report.
The members are as contained in the European Patent Office EDP file on
The European Patent Office is in no way liable for these particulars which are merely given for the purpose of information.

10-06-2013

| Patent document cited in search report | | Publication date | Patent family member(s) | | Publication date |
|---|---|---|---|---|---|
| US 2012028846 | A1 | 02-02-2012 | EP 2356072 A1<br>US 2012028846 A1<br>WO 2010078337 A1 | | 17-08-2011<br>02-02-2012<br>08-07-2010 |
| WO 2006111433 | A1 | 26-10-2006 | DE 102005018470 A1<br>WO 2006111433 A1 | | 26-10-2006<br>26-10-2006 |
| DE 10200908 | A1 | 31-07-2003 | NONE | | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **W. KUNERTH.** *Phys. Rev.,* 1922, vol. 19, 512-524
- **M.B. MILLER et al.** *J. Chem. Eng.,* 2011, vol. 56, 1565-1572
- **O. ASCHENBRENNER et al.** *Energy Environ. Sci.,* 2010, vol. 3, 1106-1113
- **O. HÖLCK.** Gas Sorption and Swelling in Glassy Polymers. *PhD thesis,* 2008
- **A. KASTURIRANGAN.** Specific interactions in carbon dioxide + polymer systems. *PhD thesis,* 2007
- **J. LIU.** Adsorption Equilibrium and Mass Transfer in Metal-Organic Frameworks and Adsorption on Carbon Surfaces in the Henry's Law Region. *PhD thesis,* 2011
- **S. BORDIGA ; L. REGLI ; F. BONINO ; E. GROPPO ; C. LAMBERTI ; B. XIAO ; P. S. WHEATLEY ; R. E. MORRIS ; A. ZECCHINA.** Adsorption properties of HKUST-1 toward hydrogen and other small molecules monitored by IR. *Phys chem chem phys,* 2007, vol. 9, 2676-2685
- **S. MARX ; W. KLEIST ; A. BAIKER.** Synthesis, structural properties, and catalytic behavior of Cu-BTC and mixed-linker Cu-BTC-PyDC in the oxidation of benzene derivatives. *J. Catal.,* 2011, vol. 281, 76-87
- **Y. C. PAN ; Y. Y. LIU ; G. F. ZENG ; L. ZHAO ; Z. P. LAI.** Rapid Synthesis of Zeolitic Imidazolate Framework-8 (ZIF-8) Nanocrystals in Aqueous System. *Chem. Commun.,* 2011, vol. 47, 2071-2073
- **M. S. Y. PARAST ; A. MORSALI.** Synthesis and characterization of porous Al(III) metal-organic framework nanoparticles as a new precursor for preparation of Al2O3 Nanoparticles. *Inorg. Chem. Commun.,* 2011, vol. 14, 645-648
- **S. BORDIGA et al.** *Phys. chem. chem. phys.,* 2007, vol. 9, 2676-2685